# EUROPÄISCHE PATENTANMELDUNG

(11) **EP 2 679 236 A1**
(43) Veröffentlichungstag der Anmeldung: **01.01.2014**
(21) Anmeldenummer: 12174285.2
(22) Anmeldetag: 29.06.2012
(51) Int. Cl.: A61K 38/12, A61P 31/00

(54) **Dosierungsanleitung für endotoxinbindende Lipopeptide**

(71) Anmelder: Zentrum für biomedizinische Technologie der Donau- Universität Krems, 3500 Krems (AT)
(72) Erfinder: Falkenhagen, Dieter, 3500 Krems (AT); Hartmann, Jens, 3511 Furth (AT); Harm, Stephan, 3508 Krustetten (AT)
(74) Vertreter: Patentanwaltskanzlei Matschnig & Forsthuber OG

(57) **Zusammenfassung**

Die Erfindung betrifft ein endotoxinbindendes Lipopeptid ausgewählt aus der Gruppe bestehend aus Polymyxinen, Polymyxin-Derivaten, Polymyxin-Analoga, deren Prodrugs und deren pharmazeutisch annehmbaren Salzen sowie eine Zubereitung zur parenteralen Verabreichung umfassend ein solches Lipopeptid, zur Prophylaxe oder zur Behandlung von Erkrankungen und Zuständen, die durch eine Endotoxinämie verursacht sind, durch i) parenterale Verabreichung eines Bolus des Lipopeptids zum Erreichen einer Lipopeptid-Serumkonzentration von 0,01 µg/ ml bis 0,8 µg/ ml und ii) Aufrechterhaltung dieser Lipopeptid-Serumkonzentration durch parenterale Verabreichung des Lipopeptids über einen vorgebbaren Zeitraum.

## Beschreibung

Die Erfindung bezieht sich auf ein endotoxinbindendes Lipopeptid ausgewählt aus der Gruppe bestehend aus Polymyxinen, Polymyxin-Derivaten, Polymyxin-Analoga, deren Prodrugs und deren pharmazeutisch annehmbaren Salzen zur Prophylaxe oder zur Behandlung von Erkrankungen und Zuständen, die durch eine Endotoxinämie verursacht sind. Die Erfindung betrifft ferner eine Zubereitung zur parenteralen Verabreichung umfassend zumindest ein solches endotoxinbindendes Lipopeptid als wirksamen Bestandteil und zumindest einen pharmazeutisch annehmbaren Träger und/ oder Hilfsstoff, zur Prophylaxe oder zur Behandlung von Erkrankungen und Zuständen, die durch eine Endotoxinämie verursacht sind.

Endotoxine sind Lipopolysaccharide (LPS) in der Zellwand gramnegativer Bakterien und werden durch Zelllyse und Zellteilung freigesetzt. In der Tat sind Lipopolysaccharide der häufigste Lipidbestandteil der äußeren Zellmembran gramnegativer Bakterien. Endotoxine sind pyrogene Substanzen und das betroffene Individuum reagiert mit einer starken Entzündungsreaktion und Fieber, wenn Endotoxine, beispielsweise im Zuge einer mikrobiellen Vergiftung, in den Körper gelangen und als Schlüsselmediatoren eine unkontrollierte Aktivierung des mononukleär-phagozytären Systems bewirken. Eine Akkumulation von Endotoxinen im Blutkreislauf infolge einer Endotoxinämie führt zu einer unkontrollierten Aktivierung der Immunzellen und einem Ungleichgewicht des Gerinnungssystems. Dies kann zu einer Sepsis führen, welche unter anderem durch hohes Fieber, niedrigen Blutdruck und, in schweren Fällen, durch Multiorganversagen gekennzeichnet ist. Eine Sepsis ist eine sehr ernstzunehmende Erkrankung; die Letalität von Individuen mit schwerer Sepsis oder septischem Schock ist je nach Schweregrad der Erkrankung ca. 30-60%. Eine Endotoxinämie infolge einer Infektion mit gram-negativen Bakterien gehört zur häufigsten Ursache für das Auftreten einer systemischen inflammatorischen Reaktion ("systemic inflammatory response syndrome", SIRS), einer Sepsis, einer schwerwiegenden Sepsis oder eines septischen Schocks und den daraus resultierenden schwerwiegenden Komplikationen. Auch Patienten mit beeinträchtigter Immunabwehr, wie z.B. Leberpatienten oder Patienten in Chemotherapie, neigen zu bakteriellen Infektionen und zeigen dadurch Symptome einer Endotoxinvergiftung. Bei akutem Leberversagen oder einer akuten Dekompensation bei chronischem Leberversagen kann es ebenfalls zu einer Endotoxinämie kommen, durch welche sich Zustände, die - biochemisch gesehen - einer Sepsis sehr ähnlich sind. Beispielsweise kann es bei Patienten mit chronischem Leberversagen zu einer akuten Dekompensation kommen. In diesem Zustand können die aus der normalen Darmflora stammenden Endotoxine die Darmbarriere überwinden und im Körper die Freisetzung von Entzündungsmediatoren stimulieren und somit einen Sepsis-ähnlichen Zustand hervorrufen.

Die Struktur eines Lipopolysaccharid-Moleküls ist dreiteilig: Ein Lipid A bildet den Bereich des Moleküls, welcher der Bakterienzelle zugewandt ist; durch das Lipid A wird das Molekül in der äußeren Membran des gramnegativen Bakteriums verankert. Das LPS-Molekül weist ferner eine mittlere, hoch konservierte Kernregion auf, welche an das Lipid A gebunden ist. Der dritte und äußerste Bereich wird durch ein O-spezifisches Polysaccharid (O-Antigen) gebildet, dessen Struktur innerhalb der verschiedenen gramnegativen Bakterien stark variieren kann. Die toxische Wirkung ist auf das Lipid A zurückzuführen, welches erst bei der Zelllyse freigesetzt wird.

Polymyxine sind antibiotische Substanzen, welche ursprünglich aus dem Bakterium Bacillus polymyxa stammen und die bereits seit langem zur Behandlung von Infektionen mit gram-negativen Bakterien bei Menschen und Tieren eingesetzt werden. Polymyxine greifen in die Zellwandstruktur ein, indem sie die Permeabilität der Zellmembran erhöhen, aufgrund dessen es zur Zelllyse kommt. Polymyxine binden nicht nur Phospholipide, sondern auch Lipopolysaccharide (Endotoxine) mit hoher Affinität. Der antibakterielle Mechanismus der Polymyxine ist beispielsweise in einer Publikation von Tony Velkov et al. eingehend beschrieben (Tony Velkov et al. 2010. Journal of Medicinal Chemistry: 53(5):1898-1916).

Aufgrund der neurotoxischen und nephrotoxischen Wirkung der Polymyxine haben nur Polymyxin B und Polymyxin E (Colistin) gewisse therapeutische Bedeutung als Antibiotikum erlangt. Bis zum heutigen Zeitpunkt sind diese beiden Polymyxine die einzigen therapeutisch zugelassenen Vertreter ihrer Substanzklasse. Polymyxin B und Colistin sind in den USA von der FDA zur parenteralen Infusion zugelassen. Polymyxin B bzw. Colistin werden seit Jahrzehnten für orale oder topische Therapieformen eingesetzt. Für eine parenterale, systemische Behandlung bei Erkrankungen und Zuständen infolge einer Infektion mit gramnegativen Bakterien kommen sie aufgrund ihrer neuro- und nephrotoxischen Nebenwirkungen allerdings nur als letzte Auswegsmöglichkeit als Antibiotikum zur therapeutischen Anwendung. Colistin scheint weniger nephrotoxisch zu sein als Polymyxin B, durch die erforderliche höhere Dosierung wird dies jedoch zumindest teilweise ausgeglichen, so dass im klinischen Alltag etwa in gleichem Ausmaß mit nephrotoxischen Reaktionen gerechnet werden kann. Ausreichende Daten zur Nephrotoxizität der beiden Antibiotika liegen aus heutiger Sicht allerdings nicht vor. Infektiologen aus New York (USA) beschreiben Nierenversagen bei 14 % von 60 Patienten, die mit Polymyxin B behandelt wurden. Ärzte in Griechenland beschreiben eine deutliche Nephrotoxizität bei der Mehrheit der Patienten, bei welchen eine renale Insuffizienz bereits bei Therapiebeginn vorlag. Bei Patienten mit normaler Funktion der Nieren wurden dagegen keine wesentlichen Veränderungen festgestellt. Eine detaillierte Übersicht über die Toxizität von Polymyxinen findet sich in einer Publikation von Falagas und Kasiakou (Falagas und Kasiakou, 2006, Critical Care 10:R27). Die Dosierung von Polymyxinen spielt folglich eine zentrale Rolle bei der Vermeidung bzw. der Minimierung toxischer Nebenwirkungen, insbesondere nephrotoxischer Nebenwirkungen.

Aufgrund des in den letzten Jahren beobachteten gehäuften Auftretens schwerwiegender Krankheitsverläufe infolge von Infektionen mit multiresistenten pathogenen Stammen, beispielsweise bei akuten Infektionen mit Stammen des Bakteriums Pseudomonas aeruginosa, werden Polymyxine trotz ihrer Toxizität notgedrungen wieder vermehrt parenteral als Antibiotikum verabreicht. Eine Bezugsquelle für Polymyxin B in Form des Sulfatsalzes von Polymxin B1 und B2 zur parenteralen Verabreichung wird derzeit von Bedford Laboratories angeboten ("Polymyxin B for Injection 500 000 Units", Hersteller: Bedford Laboratories). Gemäß Herstellerinformation erfolgt die parenterale Verabreichung intravenös, intramuskulär oder im Fall einer Meningitis intrathekal, wobei die angegebene maximale Tagesdosis in der Regel 2,5 mg/ kg Körpergewicht und Tag, aufgeteilt auf zwei bis drei Infusionen beträgt. Typischerweise liegt die Serumkonzentration von Polymyxin nach Verabreichung in einem Bereich von 1 bis 6 µg/ ml. In schwerwiegenden Fällen kann diese auch höher in einem Bereich von 6 bis 50 µg/ ml liegen. Colistin wird vorwiegend in Form von Colistin-Methansulfonat verabreicht, wobei die Serumkonzentration in einem Bereich von ca. 1 bis 3 µg/ ml liegt. Colistin (Polymyxin E) wird in gleicher Weise wie Polymyxin B eingesetzt, zumeist in höherer Dosierung.

Eine Resistenz gegenüber Polymyxin B ist eher ungewöhnlich, kann sich aber entwickeln, wenn das Antibiotikum aufgrund von Veränderungen in der äußeren Membran die Zytoplasmamembran nicht erreicht. Polymyxine sind wirksam gegen viele gramnegative Erreger, wie E. coli, Enterobacter, Klebsiella spp. und auch gegen P. aeruginosa. Proteus-Arten und S. marcescens, die normalerweise resistent sind; die Empfindlichkeit von B. fragilis ist variabel. Die minimalen Hemmkonzentrationen für E. coli liegen im Bereich von 0,04 - 3,7 mg/ l und für P. aeruginosa zwischen 1,2 und 33,3 mg/ l (Garidel und Brandenburg, 2009, Anti-Infective Agents in Medicinal Chemistry, 8:367-385).

Da die bisher in der klinischen Anwendung eingesetzten Dosierungen für Polymyxin B und Colistin bei parenteraler Verabreichung nephro- und neurotoxische Nebenwirkungen induzieren, wurden in der Vergangenheit neue Behandlungsstrategien und Therapieansätze im Zusammenhang mit der Anwendung endotoxinbindender Lipopeptide wie Polymyxin entwickelt.

Als häufig angewandte Alternative zur Verabreichung von Polymyxinen in Form eines Medikaments haben sich extrakorporale Blut- und/ oder Blutplasmareinigungsverfahren (therapeutische Apherese) unter Verwendung geeigneter Adsorptionsmaterialien etabliert. Aphereseverfahren und Adsorbermaterialien zum Eliminieren toxischer und/ oder schädlicher Stoffe aus Blut und Blutplasma sind aus dem Stand der Technik gut bekannt. Bekannte Adsorbermaterialien umfassen poröse oder faserartige Trägermaterialien, auf deren Oberflächen Polymyxin B beispielsweise kovalent oder mittels hydrophober Wechselwirkung immobilisiert ist. Bislang wurde im Zusammenhang mit derartigen Adsorbermaterialien, die in hohem Maße in der Behandlung von septischen Zuständen zum Einsatz kommen, über keine neuro- und nephrotoxischen Nebenwirkungen berichtet. Mit Polymyxin B funktionalisierte Adsorbermaterialien sind beispielsweise aus der EP 0 110 409 A1, der WO 2010/ 083545 und der WO 2011/ 160149 bekannt. Aphereseverfahren unter Verwendung geeigneter Adsorber haben jedoch den Nachteil, dass sie aufgrund des hohen technischen Aufwands, der begrenzten Verfügbarkeit der Therapieplätze und der im Vergleich zur medikamentösen Behandlung wesentlich höheren Herstellungs- und Therapiekosten nicht flächendeckend angewendet werden können und daher nur der für die intensivmedizinische Versorgung notwendige Bedarf gedeckt werden kann.

Obwohl die Letalität von Patienten mit Endotoxinämie-induzierten Erkrankungen, insbesondere Sepsis, durch die klinische Anwendung der oben genannten Polymyxin-basierten Adsorbermaterialien gesenkt werden konnte, ist die Letalität von Patienten mit schwerer Sepsis und septischen Schock trotz maximaler Therapie immer noch sehr hoch. Aus diesem Grund sowie aufgrund des immer größer werdenden Problems der Multiresistenz von Bakterien gegen Antibiotika und der damit verbundenen ansteigenden Inzidenz schwerwiegender Krankheitsverläufe besteht weiterhin ein hoher Bedarf an kostengünstigen, flächendeckenden und nebenwirkungsarmen Therapieformen. Folglich hat man sich in den letzten Jahren wieder den medikamentösen Therapieansätzen zugewandt. Beispielsweise wurde durch chemische Modifikation der Dab-Seitenketten, des zyklischen Peptidrings oder der Fettsäurekette der Polymyxin-Molekülstruktur eine Vielzahl an synthetischen Polymyxin-Analoga/ Derivate entwickelt, um Medikamente mit geringeren toxischen Nebenwirkungen bzw. einer verbesserten Endotoxinbindungseffizienz zu schaffen. Die meisten dieser Modifikationen führten jedoch zu inaktiven Verbindungen. Ferner sind für viele der beschriebenen Analoga/ Derivate keine toxikologischen Daten bekannt. Eine detaillierte Übersicht über Polymyxin-basierte Antibiotika, Analoga und Derivate ist in der Publikation von Velkov et al. gegeben (Velkov et al., 2010, Journal of Medicinal Chemistry, 53(5):1898-1916).

Die EP 2 332 965 beschreibt von natürlich vorkommenden Polymyxinen und Octapeptinen abgeleitete synthetische Peptide mit antibakteriellen Eigenschaften zur Verwendung als Antibiotikum zur Behandlung von Individuen mit einer bakteriellen Infektion sowie Verfahren zur Herstellung dieser Peptide.

Die WO 2010/ 075416 offenbart von Polymyxin, inbesondere von Polymyxin B abgeleitete chemische Verbindungen mit antibakteriellen Eigenschaften als Antibiotikum gegen eine Vielfalt an gramnegativen Bakterien. Die darin beschriebenen Verbindungen weisen eine im Vergleich zu Polymyxin B verringerte Toxizität auf.

Trotz der zahlreichen Ansätze zum Herstellen von Peptidverbindungen zu Schaffung eines Medikaments mit einer mit Polymyxin B oder Colistin vergleichbaren Endotoxinbindungseffizienz bzw. antibakteriellen Wirkung und mit deutlich verringerter Toxizität wurde bislang keine dieser Verbindungen für die klinische Anwendung zugelassen.

Es ist daher eine Aufgabe der Erfindung, die aus dem Stand der Technik bekannten Nachteile zu beseitigen bzw. zu minimieren und eine neue Dosierungsanleitung für medikamentös verabreichte endotoxinbindende Lipopeptide wie Polymyxine, Polymyxin-Analoga oder Polymyxin-Prodrugs und deren pharmazeutisch annehmbarer Salze bereitzustellen, so dass eine Prophylaxe oder eine Behandlung von Erkrankungen und Zuständen, die durch eine Endotoxinämie verursacht sind, ermöglicht wird. Es ist insbesondere eine Aufgabe der Erfindung eine neue Dosierungsanleitung für natürlich vorkommende und für die klinische Anwendung zugelassene Polymyxine wie Polymyxin B und Colistin bereitzustellen. Die neue Dosierungsanleitung soll signifikante Verbesserungen hinsichtlich der toxischen Nebenwirkungen bei unverändert hoher Wirksamkeit mit sich bringen und eine wesentlich kostengünstigere Therapiemöglichkeit für Patienten mit Endotoxinämie als die bislang für diese Zwecke eingesetzten Adsorbermaterialien darstellen.

Diese Aufgabe wird gelöst durch eine neue Dosierungsanleitung, welche gekennzeichnet ist durch i) parenterale Verabreichung eines Bolus des Lipopeptids zum Erreichen einer Lipopeptid-Serumkonzentration von 0,01µg/ ml bis 0,8 µg/ ml und ii) Aufrechterhaltung dieser Lipopeptid-Serumkonzentration durch parenterale Verabreichung des Lipopeptids über einen vorgebbaren Zeitraum.

Dank der Erfindung ist es erstmals möglich, Polymyxine bzw. Polymyxin-Derivate und Polymyxin-Analoga nicht im herkömmlichen Sinne als Antibiotikum, also zur Elimination gramnegativer Bakterien einzusetzen, sondern in deutlich geringerer Konzentration (4- bis 100-fach geringer) zur Inaktivierung der Endotoxine in Patienten mit Endotoxinämie. Eine Inaktivierung der Endotoxine bedeutet, dass deren biologische Wirkung, insbesondere auf die Ausschüttung von Entzündungsmediatoren wie Cytokinen, gehemmt oder blockiert wird. Dies hat zur Folge, dass die proinflammatorische Phase, die z.B. in der Sepsis oder bei SIRS durch gramnegative Endotoxine hervorgerufen wird, unterdrückt oder verringert wird.

Die neuartige Dosierungsanleitung ermöglicht eine parenterale Verabreichung von Polymyxinen sowie deren Analoga, Derivaten und Prodrugs, wobei nephro- und neurotoxische Nebenwirkungen umgangen werden können. Die Lipopeptid-Serumkonzentration bei der erfindungsgemäßen Dosierungsanleitung liegt somit mit einem Faktor von 4 bis 100 unter der Konzentration, die bei Anwendung der zugelassenen antibiotischen Polymyxin B-Zubereitungen zur parenteralen Verabreichung erreicht wird.

Es wurde der überraschende Sachverhalt festgestellt, dass bereits sehr geringe Serumkonzentrationen an Polymyxin B von 0,01µg/ ml bis 0,8 µg/ ml ausreichen, um Endotoxine in ihrer Aktivität zu hemmen, wobei neuro- und nephrotoxische Wirkungen auszuschließen sind. Die Wirkung dieser neuartigen Dosierungsanleitung wurde anhand von Endotoxinen aus E.coli und Pseudomonas aeruginosa gezeigt, wobei bei den Versuchen eine Endotoxin-Ausgangskonzentration von 0,5 ng/ ml gewählt wurde, also eine Konzentration, die schon einen maximalen Wert bei einer klinisch relevanten Sepsis darstellt.

Die erfindungsgemäße Dosierungsanleitung basiert auf dem festgestellten überraschenden Sachverhalt, dass bei der Untersuchung verschiedener mit Polymyxin funktionalisierter Adsorbermaterialien eine Endotoxinbindung durch Polymxin ausschließlich über eine sehr geringe Menge an desorbierten und in das Blut bzw. Blutplasma übergegangenen Polymyxin-Molekülen erfolgt. Diese überraschenden und unvorhersehbaren Ergebnisse basieren auf der Tatsache, dass nach gezieltem Waschen der Adsorbermaterialien, bei welchem desorbierbare Polymyxin-Moleküle von der Adsorberoberfläche entfernt wurden, keine Endotoxinadsorption durch die noch an der Adsorbermaterialoberfläche immobilisierten Polymyxin-Moleküle festgestellt werden konnte. Es wurde festgestellt, dass auch bei einer kovalenten Bindung von Polymyxin an die Adsorberoberfläche eine bestimmte Menge an Polymyxin-molekülen über unspezifische Kräfte gebunden wird. Diese unspezifisch gebundenen Polymyxinmoleküle können bei der therapeutischen Anwendung von der Adsorberoberfläche desorbieren und im freigesetzten Zustand im Blut oder Blutplasma des Patienten vorliegende Endotoxine unschädlich machen. Somit lässt sich zusammenfassend feststellen, dass die sehr gute Endotoxinadsorption durch Adsorbermaterialien basierend auf porösen oder faserartigen Trägermaterialien, auf deren Oberflächen Polymyxin B beispielsweise kovalent oder mittels hydrophober Wechselwirkung immobilisiert ist, ausschließlich auf eine sehr geringe vom Trägermaterial desorbierte und ins Blut bzw. Blutplasma freigesetzte Menge an freien Polymyxin-Molekülen zurückzuführen ist. Die Bindung zwischen Polymyxin und Endotoxinen erfolgt offenbar nur dann, wenn sowohl die hydrophobe als auch die positiv geladenen Aminogruppen Gruppe des Polymyxin B für die Endotoxine zugänglich sind.

Die neuartige Dosierungseinleitung, wie sie in dieser Offenbarung beschrieben wird, beruht auf diesen überraschenden Ergebnissen und definiert wesentlich geringere Serumkonzentrationen an endotoxinbindenden Lipopeptiden wie Polymyxin im Vergleich zu denjenigen, die sich bei den seit vielen Jahrzehnten durchgeführten Standardtherapien mit Polymyxin B bzw. Colistin etabliert haben.

Die Begriffe "Polymyxin" bzw. "Polymyxine" wie hierin verwendet beziehen sich auf bekannte, natürlich vorkommende chemische Verbindungen, welche ursprünglich aus dem Bakterium Bacillus polymyxa (Polymyxin B) sowie Bacillus colistinus (Polymyxin E) stammen. Die Polymyxine können entweder aus Bakterien isoliert oder auf synthetischem Weg hergestellt sein. Das aus dem Bakterium stammende Polymyxin B setzt sich aus 6 Derivaten zusammen, die mit Polymyxin B1, Polymyxin B2, Polymyxin B3, Polymyxin B4, Polymyxin B5 und Polymyxin B6 bezeichnet werden. Im Vergleich dazu setzt sich das von der FDA zur parenteralen Infusion zugelassene Polymyxin nur aus Polymyxin B1 bis B4 zusammen. Klinisch relevant sind wie erwähnt bislang nur Polmyxin Bund Polymyxin E (Colistin).

Der Begriff "Polymyxin-Derivat" bezieht sich auf eine von Polymyxin abgeleitete Verbindung, die durch Modifikation natürlich vorkommender Polymyxine erhältlich ist, beispielsweise durch chemische Modifikation der Dab-Seitenketten, des zyklischen Peptidrings oder der Fettsäurekette der Polymyxin-Molekülstruktur. Eine detaillierte Übersicht über Polymyxin-basierte Antibiotika, Analoga und Derivate ist in der Publikation von Velkov et al. beschrieben (Velkov et al., 2010, Journal of Medicinal Chemistry, 53(5):1898-1916). Ein repräsentatives Beispiel für ein Polymyxin-Derivat ist Polymyxin-Nonapeptid, ein Polymyxin B-Derivat, welchem der hydrophobe Anteil und eine Aminosäure fehlen.

Der Begriff "Polymyxin-Analogon" wie hierin verwendet bezieht sich auf eine chemische Lipopeptid-Verbindung, die strukturell einem Polymyxin ähnlich oder mit einem Polymyxin vergleichbar ist ("polymyxin-like lipopeptide") und die eine gleiche endotoxinbindende Wirkung wie Polymyxine oder eine mit Polymyxinen vergleichbare endotoxinbindende Wirkung aufweist. Ein repräsentatives Beispiel für ein solches Polymyxin-Analogon ist der Offenbarung WO 2008/ 006125 A1 zu entnehmen.

Der Begriff "Prodrug" wie hierin verwendet, bezieht sich auf eine Vorläuferverbindung des endotoxinbindenden Lipopeptids wie definiert, wobei die Vorläuferverbindung in vivo in das aktive endotoxinbindende Lipopeptid umgewandelt wird. Als repräsentative Beispiele sind die Prodrugs Colistin-Methansulfonat und Polymyxin B-Methansulfonat-Natrium zu nennen.

Der Begriff "Endotoxinämie" wird hierin für sämtliche Krankheitsbilder verwendet, bei welchen sich klinisch relevante Mengen an Endotoxinen im Blut des Patienten befinden, welche in weiterer Folge zur Induktion von Cytokinen, vorzugsweise in Krankheitsbildern wie Sepsis und SIRS, führen.

Die Dosierungsanleitung ist sowohl zur Behandlung als auch zur Prophylaxe von Erkrankungen und Zuständen, die durch eine Endotoxinämie verursacht sind, geeignet. Unter "Prophylaxe" ist eine Verabreichung des endotoxinbindenden Lipopeptids zu verstehen, wenn eine Endotoxinämie vorliegt, aber noch keine klinischen Symptome vorhanden sind. Eine prophylaktische Therapie kann besonders bei solchen Patienten indiziert sein, bei denen aufgrund ihrer Erkrankung mit dem Auftreten einer Endotoxinämie zu rechnen ist, beispielsweise bei Patienten, die an akutem Leberversagen oder an einer akuten Dekompensation bei chronischem Leberversagen leiden, so dass im Zuge der Überwachung dieser Patienten bei Auftreten einer Endotoxinämie und noch vor dem Auftreten klinischer Symptome die endotoxinbindenden Lipopeptide entsprechend der erfindungsgemäßen Dosierungsanleitung verabreicht werden.

Darüber hinaus ist bekannt, dass es beim Einsatz von Antibiotika bei einer Infektion mit gram-negativen Bakterien und durch die durch die Antibiotikagabe induzierte Zelllyse zu einer vermehrten Endotoxinausschüttung kommt bzw. kommen kann. Die Erfindung kann daher mit Vorteil als zusätzliche therapeutische oder prophylaktische Maßnahme im Rahmen einer konventionellen Behandlung von bakteriellen Infektionserkrankungen mittels Antibiotika zur Anwendung kommen, um die durch Antibiotikagabe induzierte Endotoxinausschüttung, welche in weiterer Folge zur Induktion von Cytokinen führt, abzufangen.

Der Begriff "parenterale Verabreichung" wie hierin verwendet, bezieht sich auf andere Verabreichungswege als eine enterale und topische Verabreichung und bezieht sich insbesondere auf eine Injektion bzw. Infusion, wobei die Injektion bzw. Infusion vorzugsweise, ohne darauf beschränkt zu sein, intravenös, subkutan, intramuskulär, intraarteriell oder intrathekal erfolgen kann. Eine parenterale Verabreichung hat den Vorteil, dass die zu erreichende Lipopeptid-Serumkonzentration sowohl bei der initalen Bolusgabe als auch beim Aufrechterhalten der Serumkonzentration über einen vorgebbaren Zeitraum rasch einstellbar und aufrecht erhaltbar ist. Vorzugsweise erfolgt die parenterale Verabreichung in Form einer intravenösen Infusion oder durch Infusion in das in einem extrakorporalen Blutkreislauf geführte Blut wie weiter unten im Detail beschrieben.

Die Erfindung ist sowohl für den humanmedizinischen als auch den veterinärmedizinischen Bereich einsetzbar. Der Begriff "Patient" wie hierin verwendet bezieht sich folglich auf Menschen und Tiere. Aufgrund des erhöhten Auftretens von Infektionen durch multiresistente Stamme und des damit verbundenen Bedarfs an neuen Therapieformen, ist die Erfindung besonders für den humanmedizinischen Bereich von hoher Relevanz.

Da natürlich vorkommende Polymyxine, welche ursprünglich aus dem Bakterium Bacillus polymyxa stammen, zu den am besten untersuchten Peptidantibiotika gehören und bereits seit Jahrzehnten in der Behandlung von Erkrankungen und Zuständen infolge einer Endotoxinämie zur Anwendung kommen, ist es bevorzugt, wenn das endotoxinbindende Lipopeptid ein Polymyxin ist. Besonders bevorzugt ist das Lipopeptid ausgewählt aus der Gruppe bestehend aus den bislang einzigen für die klinische Anwendung zugelassenen Polymyxinen Polymyxin B und Colistin (Polymyxin E). Am meisten bevorzugt wird jedoch Polymyxin B, da es sich für die Anwendung im humanmedizinischen Bereich am besten bewährt hat. Polymyxin B kommt vorzugsweise in Form von Polymyxin B-Sulfat zum Einsatz.

Ein weiterer Gegenstand der Erfindung ist ferner eine Zubereitung zur parenteralen Verabreichung, umfassend zumindest ein endotoxinbindendes Lipopeptid wie in dieser Offenbarung definiert als wirksamen Bestandteil und optional zumindest einen pharmazeutisch annehmbaren Träger und/ oder Hilfsstoff. Die Zubereitung kann nur eine Art eines endotoxinbindenen Lipopeptids oder eine Mischung von zwei oder mehr endotoxinbindenen Lipopeptiden umfassen, beispielsweise eine Mischung aus Polymyxin B1, B2, B3 und B4.

Ein "pharmazeutisch annehmbarer Träger und/ oder Hilfsstoff" kann jede Substanz sein, die zur Herstellung von parenteralen Verabreichungsformen wie Injektionen, Infusionslösungen und dergleichen bekannt sind. Für die Erfindung geeignete Formulierungen für Injektions- und Infusionslösungen sind unten in Beispiel 5 angeführt.

Vorzugsweise liegt die Zubereitung zur parenteralen Verabreichung in Form einer Injektionszubereitung bzw. einer Infusionszubereitung vor. Vorzugsweise liegt das endotoxinbindende Lipopeptid in Form eines lyophilisierten Pulvers zur Herstellung einer sterilen wässrigen Injektionszubereitung bzw. Infusionszubereitung vor, wobei das Pulver beispielsweise in sterilem Wasser, 5 %iger Dextroselösung, einer Ringerlösung oder einer physiologischen Natriumchloridlösung gelöst werden kann.

Das Lipopeptid liegt in der Zubereitung in gelöster Form in Schritt i) für die parenterale Verabreichung eines Bolus vorzugsweise in einer Konzentration von 5 mg/ l bis 200 mg/ l, und in Schritt ii) für die Aufrechterhaltung der Serumkonzentration vorzugsweise in einer Konzentration von 0,04 mg/ l bis 13 mg/ l, mehr bevorzugt von 0,1 mg/ l bis 7 mg/ l, am meisten bevorzugt von 0,5 mg/ l bis 4 mg/ l vor.

Vorzugsweise liegt die Lipopeptid-Serumkonzentration in einem Bereich von 0,1 µg/ ml bis 0,6 µg/ ml, vorzugsweise 0,1 µg/ ml bis 0,4 µg/ ml, am meisten bevorzugt zwischen 0,1 µg/ ml bis 0,25 µg/ ml, da bei diesen Serumkonzentrationen auch bei schwerwiegenden Krankheitsverläufen wie einer Sepsis, einer schwerer Sepsis oder einem septischen Schock eine effiziente Therapie ohne neuro- und nephrotoxische Nebenwirkungen durchführbar ist.

Die Lipopeptid-Serumkonzentration ist erfindungsgemäß in einem ersten Schritt i) wie in den Ansprüchen definiert durch eine einmalige Bolusgabe des Lipopeptids erreichbar, um die angestrebte Serumkonzentration zu erhalten. Im Rahmen dieser Offenbarung wird unter dem Begriff "Bolus" somit eine einmalige parenterale Verabreichung des endotoxinbindenden Lipopeptids in Form einer Zubereitung vorzugsweise in Form einer Injektions- oder Infusionszubereitung verstanden, wobei die Zubereitung für die Bolusgabe vorzugsweise eine höhere Konzentration an Lipopeptid als jene Zubereitung hat, die in Schritt ii) für die anschließende Aufrechterhaltung der Lipopeptid-Serumkonzentration zum Einsatz kommt.

Die Bolusgabe in Schritt i) erfolgt vorzugsweise über einen Zeitraum von zumindest 10 Minuten, mehr bevorzugt von zumindest 60 min, am meisten bevorzugt von zumindest 120 min, vorzugsweise durch parenterale Injektion oder Infusion, idealerweise nicht während einer Dialysebehandlung. Für die Ermittlung der erforderlichen Polymyxinmenge ist das Blutvolumen des Patienten zu berücksichtigen. Die Bolusgabe (Schritt i)) erfolgt vorzugsweise als Injektion, wobei beispielsweise ein einmaliger Bolus von 10 bis 250 ml der zubereiteten Injektionslösung zu Behandlungsbeginn verabreicht wird. Die Bolusgabe kann ferner mittels Infusion erfolgen.

In einem zweiten Schritt ii) wird diese mittels Bolusgabe sehr rasch eingestellte Lipopeptid-Serumkonzentration über einen vorgebbaren Zeitraum aufrechterhalten, wobei das Aufrechterhalten der Serumkonzentration vorzugsweise durch Infusion, die vorzugsweise kontinuierlich erfolgt, durchgeführt wird. Die Infusionsrate ist von der Serumhalbwertszeit für das Lipopeptid im Patienten abhängig. Beispielsweise liegt die Serumhalbwertszeit für Polymyxin B bei Patienten mit normaler Nierenfunktion typischerweise bei 13 Stunden, jene für Colistin je nach Literaturangabe bei 6 bis 7,4 Stunden. Bei gleichzeitiger Behandlung mittels eines extrakorporalen Blutreinigungsverfahrens ist wie weiter unten im Detail beschrieben auch die Clearance des jeweils eingesetzten Filters und/ oder die Clearance eines Adsorbersystems für das verabreichte Lipopeptid zu berücksichtigen.

Vorzugsweise beträgt in Schritt ii) der Zeitraum des Aufrechterhaltens der Lipopeptid-Serumkonzentration die gesamte Therapiedauer, also so lange eine Endotoxinämie vorliegt. Dieser Zeitraum kann von wenigen Stunden bis zu 2 Wochen oder auch länger andauern, wobei während dieses Zeitraums die unerwünschte Induktion von Cytokinen reduziert oder unterdrückt wird.

Eine detaillierte Beschreibung wie die angestrebte Serumkonzentration erreicht werden kann, findet sich unten in den Beispielen.

In einer Variante der Erfindung, die besonders kostengünstig und flächendeckend zum Einsatz kommen kann, erfolgt in Schritt ii) die Aufrechterhaltung der Lipopeptid-Serumkonzentration durch intravenöse Verabreichung. Bei dieser Variante wird das endotoxinbindende Lipopeptid intravenös über einen Venenzugang, vorzugsweise mittels einer Dosierpumpe, verabreicht.

Bei einer weiteren Variante erfolgt in Schritt ii) die Aufrechterhaltung der Lipopeptid-Serumkonzentration durch Infusion in das in einem extrakorporalen Blutkreislauf eines extrakorporalen Perfusionssystems geführte Blut eines Patienten an einer Position stromab eines dem extrakorporalen Blutkreislauf zugeordneten Dialysators (Dialysefilter). Diese Variante ist als zusätzlich therapeutische Maßnahme besonders bei schwerwiegenden oder lebensbedrohlichen Zuständen des Patienten (z.B. Sepsis) indiziert, die intensivmedizinische Maßnahmen in Form einer extrakorporalen Blut- und/ oder Plasmareinigung (therapeutische Apherese) erforderlich machen. Die Infusion des Lipopeptids erfolgt über eine Zuleitung in das Blut, welches im extrakorporalen Blutkreislauf zirkuliert, an einer Stelle stromab des Dialysators, also unmittelbar bevor das Blut wieder in den Patienten rückgeführt wird. Obwohl für die in Schritt i) verabreichte Bolusgabe eine direkte intravenöse Verabreichung zu bevorzugen ist, kann auch die Bolusgabe stromab des Dialysators in den extrakorporalen Blutkreislauf injiziert werden. Die Bolusgabe sollte vorzugsweise zeitlich vor der Dialysebehandlung erfolgen, die kontinuierliche Infusion gleichzeitig mit der Dialyse und an einer Position bevorzugt stromabwärts des Dialysators.

Da bei dieser Variante das endotoxinbindende Lipopeptid nicht nur vom Körper abgebaut wird, sondern auch über den Dialysator aus dem Blut entfernt wird, ist es bei der Dosierung des infundierten Lipopeptids in das im extrakorporalen Blutkreislauf geführte Blut zweckmäßig, die Lipopeptid-Clearance des Körpers und die Lipopeptid-Clearance des Dialysators zu berücksichtigen.

Ist dem extrakorporalen Perfusionssystem noch dazu eine Abreicherungseinrichtung, beispielsweise in Form einer Adsorberkartusche oder eines Plasmakreislaufes mit darin suspendierten Adsorberpartikeln, zugeordnet, so ist es günstig, wenn bei der Dosierung des infundierten Lipopeptids zusätzlich die Lipopeptid-Clearance einer dem extrakorporalen Perfusionsystem zugeordneten Abreicherungseinrichtung berücksichtigt wird. Beispielsweise ist bekannt, dass Adsorber aus einem Polystyrol-Divinylbenzol-Copolymer neben pathophysiologisch relevanten Komponenten wie Cytokinen auch Lipopeptide wie Polymyxine adsorbieren, so dass die Berücksichtigung der Lipopeptid-Clearance des Adsorbers von Vorteil für die Dosierung des infundierten Lipopeptids ist.

Die Erfindung kommt mit Vorteil zur Behandlung einer Infektion mit gram-negativen Bakterien, insbesondere zur Prophylaxe oder zur Behandlung einer systemischen inflammatorischen Reaktion (SIRS), einer Sepsis, einer schweren Sepsis oder eines septischen Schocks zur Anwendung. Repräsentative Beispiele für nach der vorliegenden Offenbarung behandelbare Krankheitsbilder sind jene, die infolge einer Infektion mit gram-negativen Bakterien auftreten und in weiterer Folge in SIRS, einer Sepsis, einer schweren Sepsis mit Multiorganversagen oder einem septischen Schock münden können.

Als repräsentative Beispiele für gram-negative Bakterien sind Escherichia spp, Haemophilus influenzae, Pseudomonas aeruginosa, Pasteurella, Enterobacter spp., Salmonella spp., Shigella spp. Besonders vorteilhaft ist die Erfindung bei gram-negativen Bakterien, für die ein vermehrtes Auftreten multiresistenter Stamme beobachtet wurde, wobei hier Pseudomonas aeruginosa als besonders relevanter Vertreter hervorzuheben ist.

Wie oben bereits angeführt, kommt bzw. kann es beim Einsatz von Antibiotika bei einer Infektion mit gram-negativen Bakterien und durch die durch die Antibiotikagabe induzierte Zelllyse zu einer vermehrten Endotoxinausschüttung kommen. Eine vermehrte Endotoxinausschüttung wurde beispielsweise für Antibiotika beschrieben, die bevorzugt an das PBP-3 ("penicillin-binding protein-3") binden, z.B. die häufig eingesetzten Antibiotika der Gruppe der Cephalosporine wie Ceftazidimin. Die Erfindung kommt daher mit Vorteil als zusätzliche therapeutische oder prophylaktische Maßnahme im Rahmen einer konventionellen Behandlung von bakteriellen Infektionserkrankungen mittels Antibiotika zur Anwendung, um die durch Antibiotikagabe induzierte Endotoxinausschüttung, welche in weiterer Folge zur Induktion von Cytokinen führt, abzufangen.

In einem weiteren Aspekt kommt die Erfindung mit Vorteil zur Prophylaxe oder zur Behandlung einer Entzündungsreaktion infolge eines akuten Leberversagens oder einer akuten Dekompensation bei chronischem Leberversagen, insbesondere einer systemischen inflammatorischen Reaktion (SIRS), einer Sepsis, einer schweren Sepsis mit Multiorganversagen oder eines septischen Schocks. Beim Patienten mit intakter Leberfunktionen werden die aus dem Darm in die Blutbahn übertretenden Endotoxine vom Retikuloendothelialen System (RES) bzw. den Kupfferschen Sternzellen durch Endozytose eliminiert. Bei Patienten mit chronischem Leberversagen kann es zu einer akuten Dekompensation kommen. In diesem Fall können Endotoxine der normalen Darmflora die Darmbarriere überwinden und daher die Leber ungehindert passieren und zu einer systemischen inflammatorischen Reaktion (SIRS), einer Sepsis, einer schweren Sepsis mit Multiorganversagen oder einem septischen Schock führen.

Die Erfindung betrifft ferner ein Verfahren zur Prophylaxe oder zur Behandlung von Erkrankungen und Zuständen, die durch eine Endotoxinämie verursacht sind, durch Verabreichen eines endotoxinbindenden Lipopeptids ausgewählt aus der Gruppe bestehend aus Polymyxinen, Polymyxin-Derivaten, Polymyxin-Analoga, deren Prodrugs und deren pharmazeutisch annehmbaren Salzen, durch i) parenterale Verabreichung eines Bolus des Lipopeptids zum Erreichen einer Lipopeptid-Serumkonzentration von 0,01 µg/ ml bis 0,8 µg/ ml und ii) Aufrechterhaltung dieser Lipopeptid-Serumkonzentration durch parenterale Verabreichung des Lipopeptids über einen vorgebbaren Zeitraum. Die oben angeführten Definitionen und Weiterbildungen sind auf das Verfahren gleichermaßen anzuwenden.

Die Erfindung wird im Folgenden anhand von nicht einschränkenden Beispielen näher erläutert.

### Beispiel 1: Endotoxin(LPS)-Inaktivierung in Abhängigkeit der Polymyxin-Konzentration anhand von Endotoxinen aus E. coli und Pseudomonas aeruginosa.

### 1.1. Ziel

Ziel dieses Versuches ist es, die Polymyxin B (PMB)-konzentrationsabhängige Endotoxininaktivierung im Plasma zu ermitteln (Batchtest I). Weiteres soll untersucht werden inwiefern diese Endotoxininaktivierung eine Hemmung der Cytokinausschüttung zu Folge hat (Batchtest II).

### 1.2. Blutspende

Einem Spender werden 9 Blutabnahmeröhrchen (zu je 9 ml) mit 5 IU Heparin gespiket abgenommen. Das Plasma wird abzentrifugiert und das Zellpellet am Rollenmischer inkubiert. Das Plasma wird mit LPS gespiket und für den Batchtest I verwendet:

### 1.3. LPS-spike, Polymyxin B-Lösungen und Batchtest I

LPS: Pseudomonas aeruginosa (L-7018 Fa. Sigma Lot: 128K4115, -70°C, a 100 ul 10-3 g/ ml (1mg/ ml))

LPS: E. coli (L-4130 Fa. Sigma Lot: 110M4086M, -70°C, a 100 µl 10-3 g/ ml (1mg/ ml))

Das LPS wird im Batch mit einer Endkonzentrationen von 0,5 ng/ ml eingesetzt. Die Ansätze werden in 3 ml pyrogenfreie Glasvials durchgeführt. Im Batchtest I werden im Zweifach-Ansatz unterschiedliche PMB-Konzentrationen (Fa. Sigma, P-1004) zugesetzt und für 60 min am Überkopf-Schüttler bei 37 °C inkubiert (siehe Tabelle 2).

Im Batchtest I werden PMB-Konzentrationen mit 0 (ohne PMB), 10, 100, 250, 500 und 1000 ng/ ml eingesetzt. Dafür werden sterile PMB-Lösungen (pyrogenfrei autoklaviert bei 121 °C, 90 min) mit folgenden Konzentrationen hergestellt (Tabelle 1):

**Tabelle 1:**

| | PMB[ng/ml] | PMB[ng/ml] im Batch (1:15) |
|---|---|---|
| PMB-Lösung A | 150 | 10 |
| PMB-Lösung B | 1500 | 100 |
| PMB-Lösung C | 3750 | 250 |
| PMB-Lösung D | 7500 | 500 |
| PMB-Lösung E | 15000 | 1000 |
| NaCl-Lösung | 0 | 0 |

### 1.4. Endotoxin-Analyse

Mit Hilfe eines Limulus Amebocyte Lysate test (LAL) von Charles River werden die Endotoxine in Form von EU/ ml gemessen.

### 1.5. Cytokinbatch (Batchtest II)

Das mit LPS und PMB gespikte Plasma wird nach dem Batchtest I auf das vom Blutspender gewonnene Zellkonzentrat im Verhältnis 1:1 zurückgeführt (siehe Tabelle 2). Für den Cytokinbatch wurden die Proben aus Batchtest I mit einer PMB-Konzentration von 0 (ohne PMB), 250, 500 und 1000 ng/ ml herangezogen. Als Kontrolle wurde eine Probe ohne LPS und mit 1000 ng/ ml PMB mitgeführt. Nach den Inkubationszeiten von 4 h und 12 h bei 37 °C am Rollenmixer (5 Umdrehungen/ min) werden Proben gezogen, abzentrifugiert und 50 µl Plasma bei -80 °C für die spätere Cytokin-Quantifizierung eingefroren. Die Versuchsdaten zum Cytokinbatch sind in der Tabelle 2 aufgelistet.

**Tabelle 2 :**

| **LPS *Pseudomonas aeruginosa*** | | | | | | | | |
|---|---|---|---|---|---|---|---|---|
| PMB [ng/ml] | PMB-Lsg | Plasma + 0,5 ng/ml LPS | Inkubation | LAL | EU/ml | Cytokin-Batch | Probe 4 h | Probe 12 h |
| 0 | 100 µl NaCl | 1400 µl | 60min | #1 | 0,333 | 1500 µl Zellenkonzentrat + | | 250 µl → 50 µl |
| 0 | 100 µl NaCl | 1400 µl | 60 min | #2 | 0,229 | 1500 µl LPS-PMB Plasma | 250 µl → 50 µl Plasma -80 C | Pasma -80°C |
| 10 | 100 µl Lsg A | 1400 µl | 60 min | #3 | 0,178 | | | |
| 10 | 100 µl Lsg A | 1400 µl | 60 min | #4 | 0,167 | | | |
| 100 | 100 µl Lsg B | 1400 µl | 60 min | #5 | 0,112 | | | |
| 100 | 100 µl Lsg B | 1400 µl | 60 min | #6 | 0,137 | | | |
| 250 | 100 µl Lsg C | 1400 µl | 60 min | #7 | 0,108 | 1500 µl Zellenkonzentrat + 1500 µl LPS-PMB Plasma | 25 µl → 50 µl Plasma -80 °C | 250 µl → 50 µl Plasma -80 °C |
| 250 | 100 µl Lsg C | 1400 µl | 60 min | #8 | 0,123 | | | |
| 500 | 100 µl Lsg D | 1400 µl | 60 min | #9 | 0,091 | 1500 µl Zellenkonzentrat + | | 250 µl → 50 µl |
| 500 | 100 µl Lsg D | 1400 µl | 60 min | #10 | 0,081 | 1500 µl LPS-PMB Plasma | 250 µl → 50 µl Plasma -80 °C | Plasma -80 °C |
| 1000 | 100 µl Lsg E | 1400 µl | 60 min | #11 | 0,062 | 1500 µl Zellenkonzentrat + | | 250 µl → 50 µl |
| 1000 | 100 µl Lsg E | 1400 µl | 60 min | #12 | 0,061 | 1500 µl LPS-PMB Plasma | 250 µl → 50 µl Plasma -80 °C | Pasma -80 °C |
| | | | | | | | | |

| **LPS** ***E**.coli* | | | | | | | | |
|---|---|---|---|---|---|---|---|---|
| PMB [ng/ml] | PMB-Lsg | Plasma + 0,5 ng/ml LPS | Inkubation | LAL | EU/ml | Cytokin-Batch | Probe 4 h | Probe 12 h |
| 0 | 100 µl NaCl | 1400 µl | 60 min | #13 | 1,8 | 1500 µl Zellenkonzentrat + | | 250 µl → 50 µl |
| 0 | 100 µl NaCl | 1400 µl | 60 min | #14 | 1,841 | 1500 µl LPS-PMB Plasma | 250 µl → 50 µl Plasma -80 °C | Plasma -80 °C |
| 10 | 100 µl Lsg A | 1400 µl | 60 min | #15 | 0,77 | | | |
| 10 | 100 µl Lsg A | 1400 µl | 60 min | #16 | 0,871 | | | |
| 100 | 100 µl Lsg B | 1400 µl | 60 min | #17 | 0,379 | | | |
| 100 | 100 µl Lsg B | 1400 µl | 60 min | #18 | 0,382 | | | |
| 250 | 100 µl Lsg C | 1400 µl | 60 min | #19 | 0,281 | 1500 µl Zellenkonzentrat + | | 250 µl → 50 µl |
| 250 | 100 µl Lsg C | 1400 µl | 60 min | #20 | 0,29 | 1500 µl LPS-PMB Plasma | 250 µl → 50 µl Plasma -80 °C | Plasma -80 °C |
| 500 | 100 µl Lsg D | 1400 µl | 60 min | #21 | 0,209 | 1500 µl Zellenkonzentrat + | | 250 µl → 50 µl |
| 500 | 100 µl Lsg D | 1400 µl | 60min | #22 | 0,209 | 1500 µl LPS-PMB Plasma | 250 µl → 50 µl Plasma -80 °C | Plasma -80 °C |
| 1000 | 100 µl Lsg E | 1400 µl | 60 min | #23 | 0,154 | 1500 µl Zellenkonzentrat + | | 250 µl → 50 µl |
| 1000 | 100 µl Lsg E | 1400 µl | 60 min | #24 | 0,16 | 1500 µl LPS-PMB Plasma | 250 µl → 50 µl Plasma -80 °C | Plasma -80 °C |

### 1.6. Ergebnisse

### Endotoxinbatch (Batchtest I):

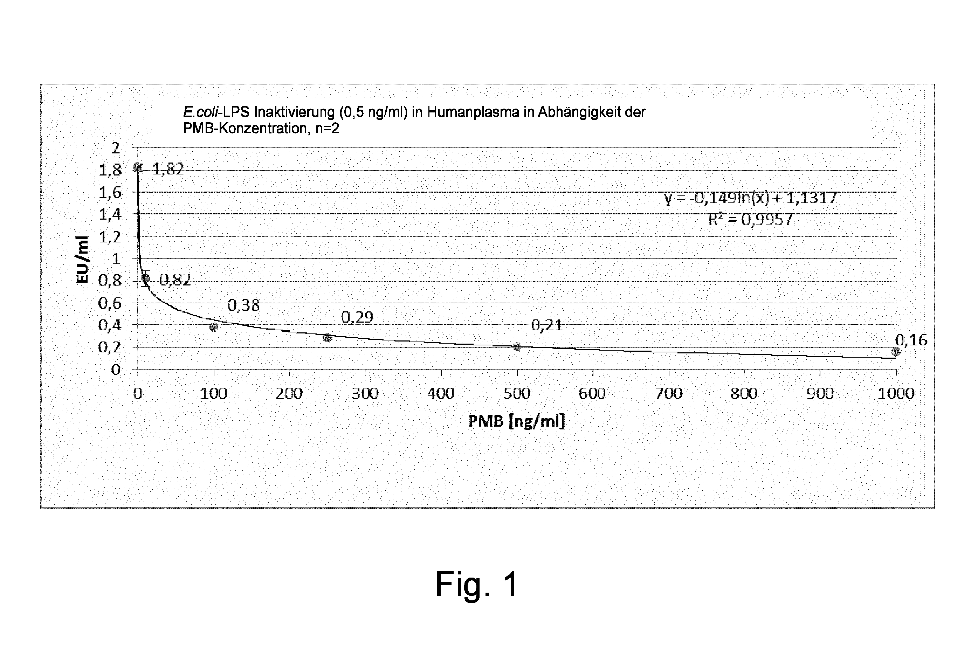

Fig. 1 zeigt die Hemmung von LPS aus E. coli in Plasma (ursprüngliche LPS-Konzentration: 0,5 ng/ ml) in Abhängigkeit der PMB-Konzentration (n=2) nach einer Inkubationszeit von 60 min.

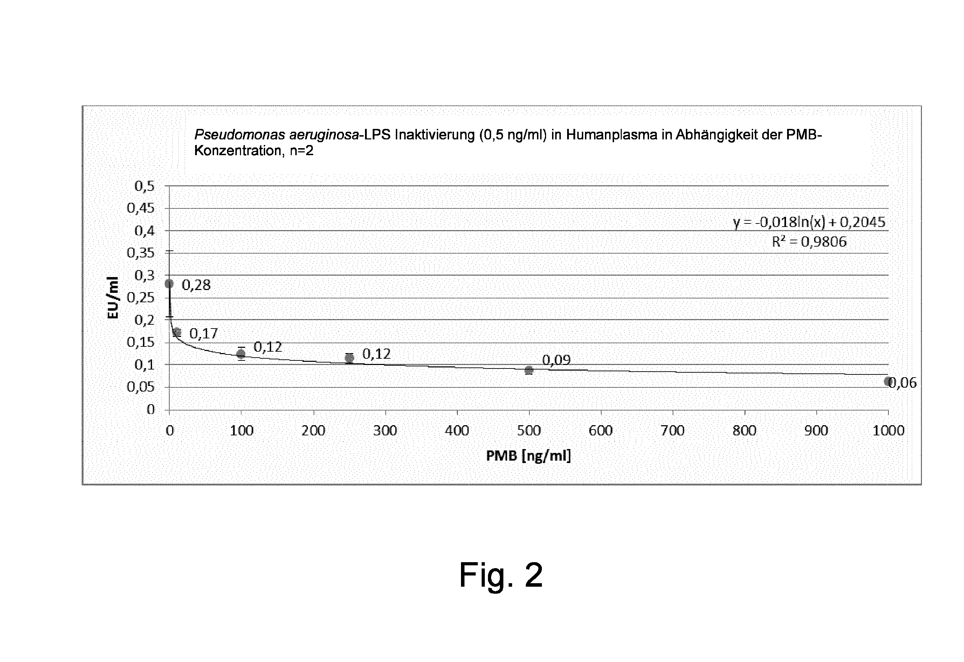

Fig. 2 zeigt die Hemmung von LPS aus Pseudomonas aeruginosa in Plasma (ursprüngliche LPS-Konzentration: 0,5 ng/ ml) in Abhängigkeit der PMB-Konzentration (n=2) nach einer Inkubationszeit von 60 min.

Die Ergebnisse zeigen deutlich, dass bereits bei einer sehr geringen PMB-Konzentration im Plasma, d.h. in einem Bereich von 50 bis 300 ng/ ml (0,05 bis 0,3 µg/ ml) eine starke Inhibierung von LPS aus E. coli und Pseudomonas aeruginosa stattfindet, wobei bei ansteigender PMB-Konzentration die LPS-Inhibierung nicht mehr signifikant zunimmt. Folglich sind bereits sehr geringe Konzentrationen an PMB ausreichend, um LPS (Endotoxine) in ihrer Aktivität zu hemmen. Bei diesen geringen Konzentrationen sind neuro- bzw. nephrotoxische Nebenwirkungen auszuschließen.

### Cytokinbatch (Batchtest II):

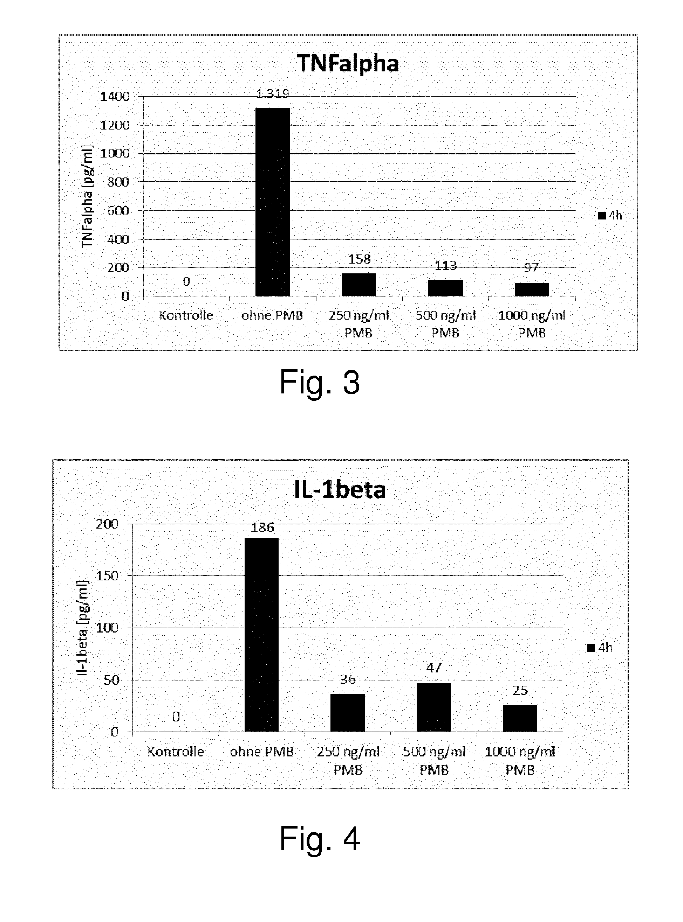

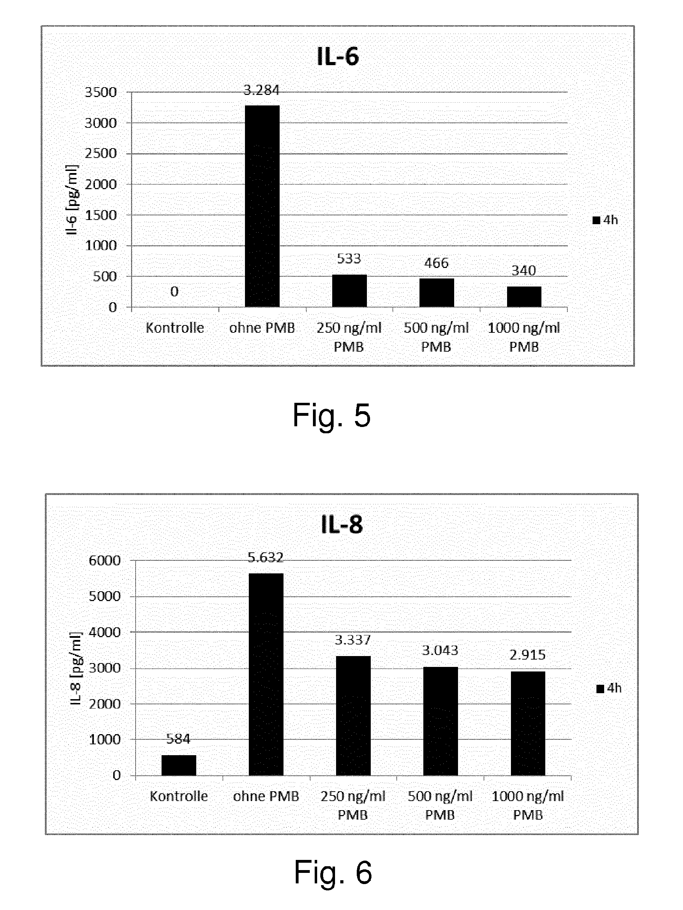

Die Ausschüttung der Cytokine TNF-alpha (Fig. 3), IL-1beta (Fig. 4), IL-6 (Fig. 5) und IL-8 (Fig. 6) durch die Blutzellen in Abhängigkeit der PMB-Konzentration (ohne PMB, 250 ng/ ml, 500 ng/ ml und 1000 ng/ ml; Kontrolle mit 1000 ng/ ml ohne LPS) in LPS (E. coli)-gespiktem Plasma nach 4 Stunden Inkubation ist in den Figuren 3 bis 6 dargestellt. Die Ergebnisse aus Batchtest II zeigen deutlich, dass bereits bei sehr geringen PMB-Konzentrationen nicht nur eine starke Inhibierung von LPS (siehe Batchtest I), sondern in Folge auch eine starke Inhibierung der Cytokinausschüttung stattfindet. Besonders ausgeprägt zeigt sich dies in der Inhibierung des Schlüsselmediators TNF-alpha (Fig. 3).

### 2. Beispiel 2: Dosierungsanleitung für Polymyxin B bei direkter intravenöser Gabe sowie Beispiele für Formulierungen für Zubereitungen zur parenteralen Verabreichung von Polymyxin B (PMB)

### 2.1. Injektionslösungen (für Bolusgabe):

### 2.1.1. Bolusgabe für eine PMB-Serumkonzentration von 100 ng/ml Plasma

Annahme: Patient mit 70 kg Körpergewicht und 60 % des Körpergewichts sind Verteilungsvolumen für PMB 42000 ml Verteilungsvolumen.

Angestrebt ist eine PMB-Serumkonzentration von 100 ng PMB/ ml Plasma es werden insgesamt 4,2 mg PMB benötigt.

Injektionslösung für eine Bolusgabe über einen Zeitraum von 60 min: 4,2 mg PMB in 100 ml physiologischer Kochsalzlösung = fertige Injektionslösung für Bolusgabe über einen Zeitraum von 60 min.

### 2.1.2. Bolusgabe für eine PMB-Serumkonzentration von 250 ng/ml Plasma

Annahme: Patient mit 70 kg Körpergewicht und 60 % des Körpergewichts sind Verteilungsvolumen für PMB 42000 ml Verteilungsvolumen.

Angestrebt ist eine PMB-Serumkonzentration von 250 ng PMB/ ml Plasma es werden insgesamt 10,5 mg PMB benötigt.

Injektionslösung für eine Bolusgabe über einen Zeitraum von 120 min: 10,5 mg PMB in 100 ml physiologischer Kochsalzlösung = fertige Injektionslösung für Bolusgabe über einen Zeitraum von 120 min.

### 2.2. Infusionslösungen (für die Aufrechterhaltung der Serumkonzentration):

Annahme: Patient mit 70 kg Verteilungsvolumen für PMB (60 % d. Körpermasse) 42000 ml Körperflüssigkeit mit 100 ng PMB/ ml 4,2 mg PMB im Verteilungsvolumen (siehe unter 2.1.1.).

Infusionslösung für eine 24 h-Infusion bei einer Serumhalbwertszeit von 6 h: Angenommene Halbwertszeit für PMB in Serum 6 h: 2,1 mg PMB pro 6 h bzw. 8,4 mg PMB/ Tag werden abgebaut 8,4 mg PMB in 1 L physiologischer Kochsalzlösung = Infusionslösung für 24 h-Infusion.

Infusionslösung für eine 24 h-Infusion bei einer Serumhalbwertszeit von 14 h: Halbwertszeit für PMB in Serum 14 Stunden: 4,2 mg PMB/ 14 h bzw. 7,2 mg PMB/ Tag werden abgebaut 7,2 mg PMB in 1 L physiologischer Kochsalzlösung = Infusionslösung für 24 h- Infusion.

### 3. Beispiel 3: Dosierungsanleitung für Polymyxin B (PMB) während einer extrakorporalen Blutreinigung (Dialyse- und Adsorptionsbehandlung) zur Aufrechterhaltung einer bereits bestehenden PMB-Serumkonzentration

Die Blutreinigungsvorrichtung nach bekannter Art umfasst einen extrakorporalen Blutkreislauf, in welchem das Blut des Patienten geführt wird und einen im extrakorporalen Blutkreislauf angeordneten Dialysator (Dialysefilter). Ferner wird im gezeigten Berechnungsbeispiel angenommen, dass der Blutreinigungsvorrichtung ein Adsorbersystem, beispielsweise in Form einer Adsorptionskartusche, zugeordnet ist, wobei das Adsorbersystem blutseitig im extrakorporalen Blutkreislauf (Hämoperfusion) oder in einem mit dem extrakorporalen Blutkreislauf über einen Plasmafilter verbundenen Plasmakreislauf (Plasma- oder fraktionierte Plasmaadsorption) verbunden sein kann.

Nachstehendes Berechnungsbeispiel setzt eine bereits bestehende PMB-Serumkonzentration voraus. Diese erfolgt durch eine Bolusgabe vor Beginn der Dialyse- und Adsorptionsbehandlung. Es können hierfür die unter Beispiel 2/ 2.1. beschriebenen Injektionslösungen verwendet werden.

Zur Berechnung der Dosierung werden die PMB-Clearance des Patientenkörpers, des Dialysators und des Adsorbersystems berücksichtigt:
- Die PMB-Dialyseclearance (CDial) kann experimentell ermittelt werden und ist vom Plasmafluß sowie vom eingesetzten Dialysefilter-Typ abhängig. Im angeführten Beispiel beträgt diese 60 ml/ min.
- Die PMB-Clearance des Adsorbers (Cads) ist vom eingesetzten Adsorbermaterial sowie vom Filtratfluss bzw. bei einer Hämoperfusion vom Blutfluss abhängig. Im angeführten Beispiel beträgt diese 45 ml/ min.
- Die PMB-Patientenclearance wurde im angeführten Beispiel aus der Halbwertszeit für PMB von 13,6 ermittelt und beträgt 36 ml/ min.

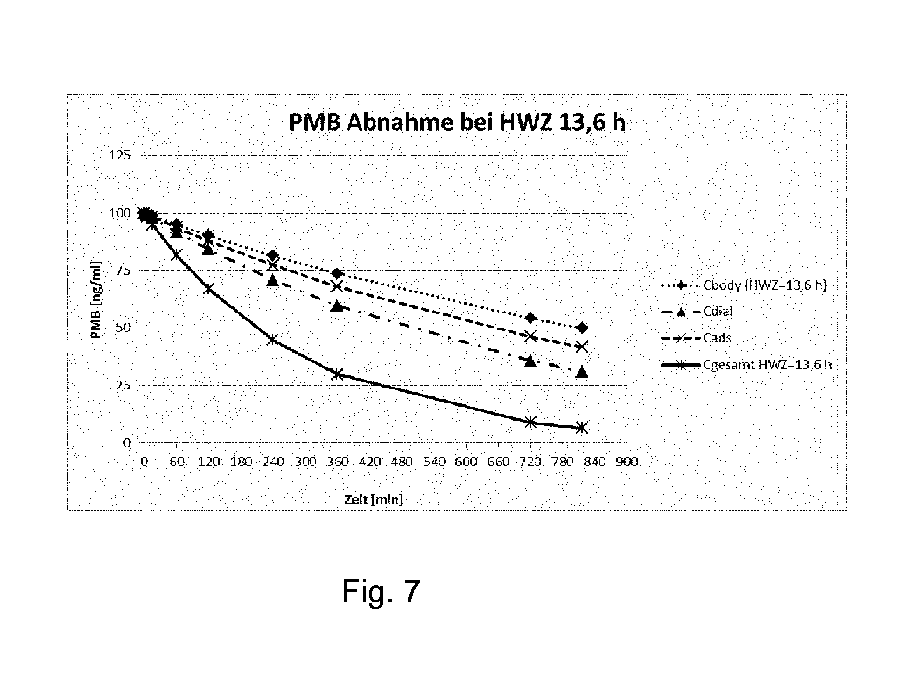

Aus den einzelnen PMB-Clearance-Raten ergibt sich durch Addition die PMB-Gesamtclearance (Cgesamt). Die daraus resultierende Abnahme des PMB ist in Fig. 7 dargestellt. Der in Fig. 7 ersichtliche negative Anstieg der PMB-Abnahme (Cgesamt) zu einem bestimmten Zeitpunkt entspricht der notwendigen PMB-Infusion, um die PMB-Serumkonzentration des zugehörigen Zeitpunkts aufrecht zu erhalten.

Für das angeführte Beispiel ergeben sich folgende Infusionsraten:
- 0,84 mg PMB/ h während der Behandlung mit Dialyse und Adsorption
- 0,21 mg PMB/ h ohne Dialysebehandlung und Adsorption

Daraus ergeben sich während 24 Stunden bei 6-stündiger extrakorporaler Behandlung folgende zu infundierende PMB-Mengen:

| | |
|---|---|
| 6 Stunden Behandlung mit Dialyse und Adsorption: | 5,1 mg |
| 18 Stunden nur PMB Infusion (ohne Dialyse und Adsorption): | 3,9 mg |
| Summe infundierte PMB-Menge während 24 Stunden: | 9,0 mg |

## Patentansprüche

1. Endotoxinbindendes Lipopeptid ausgewählt aus der Gruppe bestehend aus Polymyxinen, Polymyxin-Derivaten, Polymyxin-Analoga, deren Prodrugs und deren pharmazeutisch annehmbaren Salzen zur Prophylaxe oder zur Behandlung von Erkrankungen und Zuständen, die durch eine Endotoxinämie verursacht sind, durch
i) parenterale Verabreichung eines Bolus des Lipopeptids zum Erreichen einer Lipopeptid-Serumkonzentration von 0,01 µg/ ml bis 0,8 µg/ ml und
ii) Aufrechterhaltung dieser Lipopeptid-Serumkonzentration durch parenterale Verabreichung des Lipopeptids über einen vorgebbaren Zeitraum.

2. Endotoxinbindendes Lipopeptid nach Anspruch 1, wobei das Lipopeptid ein Polymyxin ist.

3. Endotoxinbindendes Lipopeptid nach Anspruch 2, wobei das Lipopeptid ausgewählt ist aus der Gruppe bestehend aus Polymyxin B und Colistin.

4. Endotoxinbindendes Lipopeptid nach Anspruch 3, wobei das Lipopeptid Polymyxin B ist.

5. Zubereitung zur parenteralen Verabreichung umfassend zumindest ein endotoxinbindendes Lipopeptid nach einem der Ansprüche 1 bis 4 als wirksamen Bestandteil und optional zumindest einen pharmazeutisch annehmbaren Träger und/ oder Hilfsstoff, zur Prophylaxe oder zur Behandlung von Erkrankungen und Zuständen, die durch eine Endotoxinämie verursacht sind, durch
i) parenterale Verabreichung eines Bolus des Lipopeptids zum Erreichen einer Lipopeptid-Serumkonzentration von 0,01µg/ ml bis 0,8 µg/ ml und
ii) Aufrechterhaltung dieser Lipopeptid-Serumkonzentration durch parenterale Verabreichung des Lipopeptids über einen vorgebbaren Zeitraum.

6. Zubereitung zur parenteralen Verabreichung nach Anspruch 5 in Form einer Injektionszubereitung bzw. einer Infusionszubereitung.

7. Zubereitung zur parenteralen Verabreichung nach Anspruch 5 oder 6, wobei das Lipopeptid in der Zubereitung in gelöster Form in i) für die parenterale Verabreichung des Bolus in einer Konzentration von 5 mg/ l bis 200 mg/ l vorliegt und in ii) für die Aufrechterhaltung der Serumkonzentration in einer Konzentration von 0,04 mg/ l bis 13 mg/ l, vorzugsweise von 0,1 mg/ l bis 7 mg/ l, am meisten bevorzugt von 0,5 mg/ l bis 4 mg/ l vorliegt.

8. Endotoxinbindendes Lipopeptid nach einem der Ansprüche 1 bis 4 bzw. Zubereitung zur parenteralen Verabreichung nach einem der Ansprüche 5 bis 7, wobei in i) der Zeitraum für die parenterale Verabreichung des Bolus zumindest 10 Minuten, vorzugsweise zumindest 60 min, am meisten bevorzugt zumindest 120 min beträgt.

9. Endotoxinbindendes Lipopeptid nach einem der Ansprüche 1 bis 4 und 8 bzw. Zubereitung zur parenteralen Verabreichung nach einem der Ansprüche 5 bis 8, wobei die Lipopeptid-Serumkonzentration in einem Bereich von 0,1 µg/ ml bis 0,6 µg/ ml, vorzugsweise 0,1 µg/ ml bis 0,4 µg/ ml, am meisten bevorzugt zwischen 0,1 µg/ ml bis 0,25 µg/ ml liegt.

10. Endotoxinbindendes Lipopeptid nach einem der Ansprüche 1 bis 4 und 8 bis 9 bzw. Zubereitung zur parenteralen Verabreichung nach einem der Ansprüche 5 bis 9, wobei in ii) die Aufrechterhaltung der Lipopeptid-Serumkonzentration durch intravenöse Verabreichung erfolgt.

11. Endotoxinbindendes Lipopeptid nach einem der Ansprüche 1 bis 4 und 8 bis 9 bzw. Zubereitung zur parenteralen Verabreichung nach einem der Ansprüche 5 bis 9, wobei in ii) die Aufrechterhaltung der Lipopeptid-Serumkonzentration durch Infusion in das in einem extrakorporalen Blutkreislauf eines extrakorporalen Perfusionssystems geführte Blut eines Patienten an einer Position stromab eines dem extrakorporalen Blutkreislauf zugeordneten Dialysators erfolgt.

12. Endotoxinbindendes Lipopeptid nach Anspruch 11 bzw. Zubereitung zur parenteralen Verabreichung nach Anspruch 11, wobei bei der Dosierung des infundierten Lipopeptids in das im extrakorporalen Blutkreislauf geführte Blut die Lipopeptid-Clearance des Körpers und die Lipopeptid-Clearance des Dialysators berücksichtigt werden.

13. Endotoxinbindendes Lipopeptid nach Anspruch 12 bzw. Zubereitung zur parenteralen Verabreichung nach Anspruch 12, wobei bei der Dosierung des infundierten Lipopeptids zusätzlich die Lipopeptid-Clearance einer dem extrakorporalen Perfusionsystem zugeordneten Abreicherungseinrichtung berücksichtigt wird.

14. Endotoxinbindendes Lipopeptid nach einem der Ansprüche 1 bis 4 und 8 bis 13 bzw. Zubereitung zur parenteralen Verabreichung nach einem der Ansprüche 5 bis 13, zur Behandlung einer Infektion mit gram-negativen Bakterien, insbesondere zur Prophylaxe oder zur Behandlung einer systemischen inflammatorischen Reaktion (SIRS), einer Sepsis, einer schweren Sepsis oder eines septischen Schocks.

15. Endotoxinbindendes Lipopeptid nach einem der Ansprüche 1 bis 4 und 8 bis 13 bzw. Zubereitung zur parenteralen Verabreichung nach einem der Ansprüche 5 bis 13, zur Prophylaxe oder zur Behandlung einer Entzündungsreaktion infolge eines akuten Leberversagens oder einer akuten Dekompensation bei chronischem Leberversagen, insbesondere einer systemischen inflammatorischen Reaktion (SIRS), einer Sepsis, einer schweren Sepsis oder eines septischen Schocks.
